# EUROPEAN PATENT SPECIFICATION

(11) **EP 4 311 853 B1**
(45) Date of publication and mention of the grant of the patent: **31.07.2024**
(21) Application number: 23163256.3
(22) Date of filing: 21.03.2023
(51) Int. Cl.: C10M 175/00, C11C 3/00

(54) **PROCESS FOR THE PRODUCTION OF SOLVENTS AND/OR LUBRICANTS FROM RENEWABLE SOURCES**
VERFAHREN ZUR HERSTELLUNG VON LÖSUNGSMITTELN UND/ODER SCHMIERSTOFFEN AUS ERNEUERBAREN QUELLEN.
PROCÉDÉ POUR LA PRODUCTION DES SOLVANTS ET/OU DES LUBRIFIANTS À BASE DES SOURCES RENOUVELABLE

(30) Priority: 27.07.2022 IT 202200015849
(43) Date of publication of application: 31.01.2024
(73) Proprietor: Itelyum Regeneration S.p.A., 26854 Pieve Fissiraga (LO) (IT)
(72) Inventor: DI SERIO, Martino, 26854 Pieve Fissiraga (LO) (IT); GALLO, Francesco, 26854 Pieve Fissiraga (LO) (IT); FORTUNATO, Michele Emanuele, 26854 Pieve Fissiraga (LO) (IT); TADDEO, Francesco, 26854 Pieve Fissiraga (LO) (IT); VITIELLO, Rosa, 26854 Pieve Fissiraga (LO) (IT); TESSER, Riccardo, 26854 Pieve Fissiraga (LO) (IT)
(74) Representative: Vatti, Francesco Paolo

(56) References cited:
- WO-A1-2021/149025
- US-A1- 2009 307 966

## Description

The present invention relates to a process for the production of solvents and/or lubricants from renewable sources, mainly starting from used fats and oils, also from the food ones.

Since the ancient times, the use of lubricants for machines used in major industrial productions has been known. Lubricants, typically liquid oils, or solid fats, have the power to promote the sliding of mechanical parts in reciprocal motion, reducing the friction that is generated, reducing energy consumption related to the functioning of the machines themselves, keeping a lower temperature of the parts in motion, in order to prevent melting and/or sintering of some parts, mainly made of metal or ceramic and consequent seizing, additionally reducing the wear of the components.

In -mainly chemical- industry, solvents are broadly used, which serve to dissolve and transport solid substances, promote reactions, making all the species involved available, remove foreign substances and/or pollutants, cleanse and purify and more.

For most lubricants and solvents, the most used raw material is oil, and solvents and lubricants are often hydrocarbons or derivatives thereof. Nevertheless, oil is a resource that sooner or later will be consumed -mainly because of the current rates of its use- and the process of depletion is extremely facilitated by its use, in addition to that as a raw material, as a source of energy, a use per se much less interesting than that as a raw material, but for which, unfortunately, a valuable alternative has not been found, yet. For this reason, there are many broad-range pieces of research, aimed at tracking down renewable sources. In addition to that for energy, such sources sometimes find employment also as raw materials, so as to become synergistic with the storage and extension of the life of oilfields. These employments constitute the basis of the so-called "circular economy."

A further problem is represented by wastes that, in an industrialised and consumerist civilisation such as the present one, are increasingly growing and risk bringing pollution and soiling to absolutely unacceptable levels. Among the attempts that are ongoing to alleviate the problem of wastes there is that of finding disposal processes that, instead of leading to destruction or confinement of wastes (for example, in landfills), lead to the obtainment of usable, and therefore marketable, products. This route allows those who transform wastes to be paid both by who produces wastes and wants to dispose of them and by who purchases the products, taking charge of their conversion. Conversely, disposal costs are reduced and also those of production are decreased, allowing a reduction in prices and an increase in profits. Under this perspective, also wastes can be considered renewable raw materials.

Combining these two aspects, it is possible to obtain lubricants and/or solvents from renewable sources or from waste, which is fully part of said circular economy.

In this view, several regeneration processes for waste oils and surface-active substances were set up, to obtain lubricant oils. So, Italian patent application No. 102021000033152, by the Applicant and Isuschem, relates to a process for the production of lubricant oils from renewable sources and waste, starting from soaps, used cooking oil (UCO), animal fats, refined used cooking oil (RUCO), used lubricants from renewable sources, which provides a soap acidification step and a hydrolysis step of the other waste to be regenerated to obtain fatty acids. Products from these two steps are then reacted with monoalcohols and polyalcohols, to esterify them to obtain lubricant oils and solvents. In this process, purification from the catalyst and residual glycerides of the fatty acids to be esterified occurs mainly by distillation. Distillation of fatty acids is a process that displays some difficulties. In particular, it must be carried out in vacuo and entails very high energy costs, therefore it would be desirable to be able to obtain a valuable purification of lubricant and solvent products through another route.

US 2009/0 307 966 discloses a method for the production of biodiesel using heterogeneous catalysis. The catalytic system serves both for the esterification of free fatty acids and for the transesterification of the triglycerides present in the fed oil. The catalytic system includes zinc oxide and a second metal oxide, preferably lanthanium oxide.

WO 2021/149 025 discloses a catalytic esterification process for the synthesis of an ester and a fatty acid in the presence of zinc oxide. The process includes esterification of fatty acids with mono- and poly-alcohols.

Before Italian patent No. 102021000033152, it had been found that zinc oxide could catalyse both the hydrolysis reaction of wastes to give fatty acids and the esterification reaction of the latter. Based on common knowledge, zinc oxide used as a catalyst should, at the end of the reaction, precipitate and separate from the reaction components. Willing to confirm the truthfulness of this observation, the inventors of the present invention have carried out experiments that have shown that such precipitation actually does not occur, supposedly for the presence, in the fatty acid phase, of glyceride residues that keep zinc oxide in solution. Based on their results, said inventors have obtained the present invention.

The problem on which the invention is based is that of proposing a process for the production of solvents and/or lubricants starting from renewable sources, overcoming the disadvantages mentioned, and mainly allowing to avoid the distillation of fatty acids, then significantly reducing energy consumption. This object is achieved through a process for the production of solvents and/or lubricants from renewable sources, comprising the steps of fat hydrolysis and an esterification reaction of fatty acids with alcohols in the presence of zinc oxide, characterised in a liquid substance in which zinc oxide is not capable to dissolve to the product of the fatty acid esterification reaction, generally called non-solvent, is added, obtaining a precipitate that is separated from the product and used as a catalyst in the previous hydrolysis step of the fats used as a raw material. Dependent claims describe preferred characteristics of the invention.

Further characteristics and advantages of the invention anyway will be better apparent from the following detailed description of a preferred embodiment, given only by exemplary and not limiting way and illustrated by the only enclosed figure, which represents a scheme of the process according to the present invention. In the description, used fatty acids, fats and surfactants are mentioned, but, even if with less advantage, virgin raw materials could be used instead of waste.

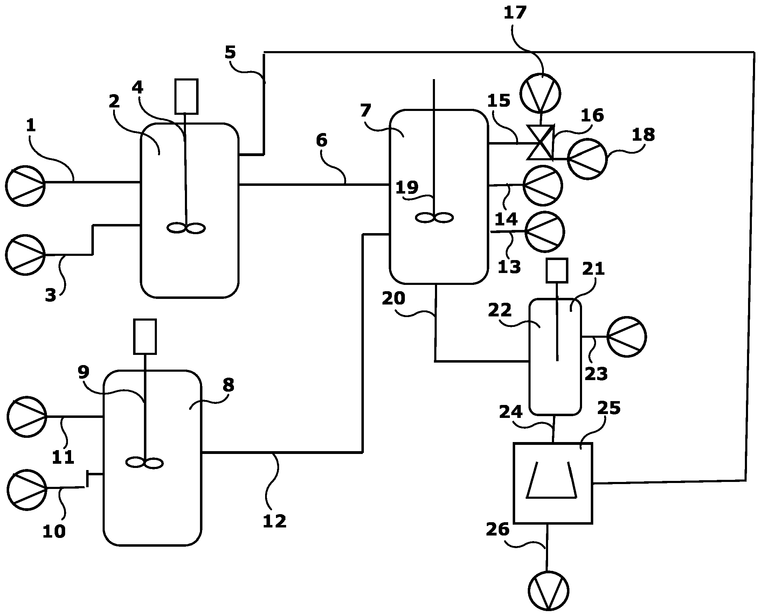

The attached figure and the following description are related to the application of the present invention to a process similar to that described and claimed in the Italian application No. IT102021000033152; however, the process according to the present invention can be applied also to other types of processes, provided that they comprise hydrolysis reactions of fats and esterification of fatty acids. As mentioned, the present invention relates to a process for the production of lubricant oils and solvents starting from virgin or waste renewable sources, such as fats and surfactants.

As observed in the attached figure, feed 1 brings to reactor 2 used cooking oils that are wastes that may generate the so-called "fatbergs", which are bodies of fat and surface-active materials that sometimes clog the sewage system, while feed 3 brings water. Based on an alternative embodiment, feed 1 can also bring waste biolubricants. Reactor 2 is provided with a stirrer 4, that can have blades as illustrated, but it can also be of any other known type. A further feed 5 feeds a catalyst (that will be described further below). From the reactor, flow 6 exits feeding reactor 7.

Another reactor 8, equipped with a stirrer 9, with blades or of another type, is fed with sulphuric acid from flow 10 and waste soaps from flow 11. From reactor 8 a flow 12 exits, feeding reactor 7 already mentioned above.

Reactor 7, in addition to flow 6 and flow 12, also receives flow 13, containing zinc oxide, flow 14, containing waste fatty acids and flow 15, coming from a three-way valve 16, where two flows merge, one, 17, containing 2-ethyl-hexanol and the other, 18, containing polyalcohols. Reactor 7 is equipped with a stirrer 19, with blade as illustrated or of a different type.

From reactor 7, a flow 20 exits, feeding reactor 21 with a stirrer 22. Also flow 23 arrives to reactor 21, feeding a non-solvent. From reactor 21 a flow 24 exits feeding a separator 25, from which a product flow 26 and flow 5, which is fed into reactor 2, exit.

Hereinafter, the process according to the present invention is described, always based on the attached figure.

In reactor 2 a hydrolysis reaction between used cooking oils and optionally lubricants derived from used renewable sources occurs. As a catalyst for the process, according to the present invention the material carried by flow 5 is used, coming from separator 25, downstream from reactor 21 and which will be discussed more thoroughly later. Generally, flows are adjusted to proceed with a weight ratio oils/water/catalyst comprised between 100:40:1 and 200:50:5. A preferred embodiment, leading to the best results, provides a ratio of 150:45:3. Temperature ranges from a minimum of 170°C to a maximum of 250°C; preferably, temperature ranges from 200 to 210°C. An operation pressure comprised between 10 and 30 bar, preferably between 13 and 20 bar is used, with a reaction time between 3 and 10 hours, preferably between 5 and 8 hours, continuously, semi-continuously or batchwise.

Reactor 2 outputs, in flow 6, a mixture of water, and optionally raw glycerol -that can be advantageously used as a base for cosmetics- and/or polyalcohols, that can be used for other manufactures -in addition to the esterification step of the present process that will be disclosed later- and, anyway, constitute one of the by-products of this process, and fatty acids. These materials are separated in a way known as such, for example in a separator (not shown in the figures for clarity'sake) that separates in the flow water and possible raw glycerol and polyalcohols and in flow 6, containing fatty acids. The particular catalyst used in the present process allows to avoid distillation steps for the fatty acids, operationally complicated and burdensome from the point of view of energy consumption. In this way, flow 6 can be fed directly in reactor 7.

According to a preferred embodiment, the process according to the present invention also provides an acidification step of waste soaps and surfactants.

In the same reactor 7 flow 12 arrives, which comes from reactor 8, in which waste soaps -optionally including reaction products of waste mineral oils with a strong base- fed in 11, are acidified with sulfuric acid fed in 12, as said previously. This operation may serve to eliminate surfactants from domestic wastewaters. In reactor 8, sulphuric acid/soap mixture has a weight ratio ranging from 1:3 to 3:1. Preferably, such ratio is 2:1. As a sulphuric acid, an aqueous solution of 70 g/l concentration is generally used, which provides a correct acidity degree, without damaging the plant though. Reaction temperature ranges between 50 and 150°C, preferably between 80 and 120°C. A particularly preferred temperature is 90°C. Preferably, the acidification reaction is carried out in an inert atmosphere, advantageously under nitrogen, to prevent side reactions that would reduce the yield of the process. Reaction time ranges between 0.5 and 4 hours, preferably between 1 and 2 hours. Outflow 12 contains fatty acids, which feed reactor 7, where flow 6 also merges and -it contains fatty acids too-, which was previously mentioned. Optionally, it can be envisaged that flow 12 is fed, before arriving at reactor 7, into a decanter tank, not shown in the figures. The flow leaving the decanter tank contains water and salts, which therefore represent the only actual waste of the process according to the present invention and are not environmentally dangerous.

Advantageously, when soaps obtained by alkalinisation of waste oils with a strong base are fed, a preliminary treatment thereof with hexane and following distillation can be envisaged, then obtaining the removal of residual oils, with a purer soap, to obtain better quality products -solvents and lubricants-.

In reactor 7, in addition to fatty acids arriving through pipes 6 and 12, also a first alcohol arrives, usually a monoalcohol, for example 2-ethyl-hexanol, fed in 17, and/or a second alcohol, usually a polyalcohol, for example trimethylolpropane, fed in 18, passing through the three-way valve 16 and the pipe 15; optionally other fatty acids, fed in 14; and zinc oxide, fed in 13. The optional addition of other waste fatty acids in 14 is useful, based on a preferred embodiment, to adjust the viscosity of the final products. Preferably, the esterification reaction of the fatty acids is carried out by reacting fatty acids with an alcohol selected in the group consisting of 2-ethyl-hexanol, ethylene glycol, propylene glycol, 1,4-butylene glycol, 1,3-butylene glycol, neopentyl glycol, trimethylolpropane, pentaerythritol; on their side, fatty acids that are subjected to esterification reaction have a number of carbon atoms ranging between 8 and 22, which are those leading to the most sought-after lubricants and solvents on the market.

In reactor 7, under shaking of stirrer 19, esterification of fatty acids is carried out. Zinc oxide -as it was noted in the introduction- has a catalytical function. Preferably, weight ratios of fatty acids/alcohol/catalyst range from 100:5:1 to 100:30:5; even preferably, they range from 100:10:1 to 100:20:3. Temperature ranges from 150 to 300°C, preferably from 180 to 220°C. Reaction time ranges from 2 to 8 hours, preferably from 4 to 6 hours. Since after the hydrolysis reaction catalyst remains mainly in the fatty acid phase, flow 6 already feeds to reactor 7 almost all the zinc oxide required for the esterification catalysis. Flow 13 feeds zinc oxide only to start the plant, to compensate for the unavoidable losses and, if the waste soap acidification in reactor 8 is foreseen, to take into account the fatty acids arriving with flow 12 into reactor 7, keeping unchanged the most suitable ratio to obtain the intended results.

Both natural alcohols, such as ethanol and butanediol, and synthetic alcohols, such as 2-ethyl-hexanol, trimethylolpropane (TMP) and pentaerythritol can be used.

A flow 20 exits from reactor 7, which is fed into reactor 21. In addition to flow 20, reactor 21 is fed with a non-solvent in 23. All liquid substances in which zinc oxide is not capable to dissolve can be used as a non-solvent. Particularly useful are water, alcohols, such as methanol, ethanol, propanol or butanol, and mixtures thereof. Said non-solvent can be added in a stoichiometric amount or in excess with respect to the stoichiometric. To maximise the removal of catalyst from the product exiting in 26, the treatment with the non-solvent can be repeated several times, for example resorting to recycling of a part of flow 24 into reactor 21 or several reactors 21 in series. In this way, in any case, the formation of a precipitate occurs. From reactor 21 a flow 24 exits which contains lubricants and product solvents and a solid. Flow 24 is fed into the separator 25, from which a product flow 26 exits, if needed to be further purified, in a way known as such before marketing, and flow 5, containing a solid. This solid is used as a catalyst for the hydrolysis reaction of oils, in reactor 2.

Catalyst exiting in flow 5, according to a preferred embodiment, can also be sent into flow 13 and be used as a catalyst also in the esterification reaction of fatty acids. In some embodiments, the present invention allows not adding zinc oxide once the starting step of the plant is completed, indeed recycling all the catalysts.

As it was observed, the present invention allows obtaining from some food and lubrication wastes, products with high added value, usable in the industry as solvents or for the production of lubricants. There are also by-products, with a lower added value, as glycerol and polyalcohols, but anyway useful and marketable, recovering almost everything that had been discarded.

In this way, with a good corresponding waste collection, said products can be obtained at relatively moderate costs, relieving problems such as that of fatbergs and others of the same nature, and additionally reducing the consumption of blank raw materials. Moreover, there is a remarkable energy saving, bound to the elimination of the fractioned distillation steps, possibly under vacuum, owed to the quantitative recovery of the catalyst that the present invention enables.

The following examples better illustrate the results that can be obtained thanks to the present invention.

### Example 1 - Comparative examples.

### Esterification of nonanoic acid (AN) with trimethylolpropane (TMP) .

An esterification reaction of nonanoic acid (AN) with trimethylolpropane (TMP) was carried out inside a 500 ml glass flask, loading 150 g of acid therein, alcohol in a stoichiometric ratio and catalyst (ZnO) in an amount equal to 2% mol/mol with respect to the acid. The flask was immersed in an oil bath and warmed to a temperature of 180°C. The reaction was carried out for the first 4 hours under vacuum, until a degree of conversion higher than 90% was reached. Subsequently, a flow of nitrogen to reach conversion values at least equal to 98% was used. After allowing the system to cool down, the catalyst was centrifuged off. The following analyses on the final product were performed (table 1).

**Table 1. Characterisation of ester from nonanoic acid (AN) and trimethylolpropane (TMP).**

| **Parameter** | **Ester** | |
|---|---|---|
| **Acid number [mg KOH/g sample]** | | 0.97 |
| **Saponification number [mg KOH/g sample]** | | 310.59 |
| **Viscosity @ 40°C [mPa s]** | | 21.53 |
| **Viscosity @ 100°C [mPa s**] | | 5.1 |
| **Zn [ppm]** | | 2.29 |

### Esterification of oleic acid (AO) with trimethylolpropane (TMP)

The esterification reaction of oleic acid (AO) with trimethylolpropane (TMP) was carried out inside a 500 ml glass flask, loading 150 g of acid therein, alcohol in a stoichiometric ratio and catalyst (ZnO) in an amount equal to 2% mol/mol with respect to the acid. The flask was immersed in an oil bath and brought to a temperature of 180°C. The mixture was reacted for the first 4 hours under vacuum, until a degree of conversion higher than 90% was reached. Subsequently, the reaction was carried out under a nitrogen stream, until a final conversion at least equal to 98% was reached. After allowing the system to cool down, the catalyst was centrifuged off. The following analyses were performed on the final product (table 2) .

**Table 2. Characterisation of ester from oleic acid (AO) and trimethylolpropane (TMP).**

| **Parameter** | **Ester** |
|---|---|
| **Acid number [mg KOH/g sample]** | 1.02 |
| **Iodine number [g I₂/100g sample]** | 90.56 |
| **Saponification number [mg KOH/g sample]** | 198.25 |
| **Viscosity @ 40°C [mPa s]** | 51 |
| **Viscosity @ 100°C [mPa s]** | 9.65 |
| **Zn [ppm]** | 15.6 |

### Example 2 - Hydrolysis of used frying oil

Hydrolysis reactions were carried out, loading 450 g of used frying oil in an autoclave, 135 g of water corresponding to 30% by weight with respect to the oil and 9 g of zinc oxide as a catalyst in an amount corresponding to 2% by weight with respect to the oil. Heating was started, up to the temperature of 200°C, reaching a maximum pressure of 13,3 bar. The reaction had a total duration of 7 hours, at the end of which the system was cooled to a temperature of about 60°C and the fatty acid products were separated from the aqueous phase containing glycerol. Waste oil as a raw material and respective fatty acid products were characterised, obtaining the results reported in table 3.

**Table 3. Characterisation of waste frying oil and fatty acids.**

| **Parameter** | **Used frying oil** | **Acid phase** |
|---|---|---|
| **Acid number [mg KOH/g sample]** | 0.29 | 152.97 |
| **Iodine number [g I₂/100g sample]** | 83.5 | 91.05 |

### Example 3 - Esterification of fatty acids from example 2 with TMP

Fatty acids obtained from the hydrolysis described in example 2, in an amount equal to 424.2 g, were used in an esterification reaction with TMP (51.72 g), in a stoichiometric ratio, without adding further ZnO as a catalyst. The system was heated until a temperature of 180°C was reached (15 min were necessary for the heating step) and this was maintained for the first 30 min (without using vacuum). Subsequently, it was increased until 200°C and remained so for 3 h (by using vacuum) and eventually was maintained at 220°C for 1.5 h. The reaction had a total duration of 5 hours.

At the end of the reaction, in contrast with the tests carried out in example 1, there was no precipitation of catalyst, in table 4 the results of the analyses carried out.

**Table 4. Characterisation of ester.**

| **Parameter** | **Ester** |
|---|---|
| **Acid number before separation of ZnO [mg KOH/g sample]** | 8.12 |
| **Iodine number [g I₂/100g sample]** | 101.97 |
| **Saponification number [mg KOH/g sample]** | 178.06 |
| **Viscosity @ 40°C [mPa s]** | 55.73 |
| **Viscosity @ 100°C [mPa s]** | 10.54 |

### Example 4 - Treatment with alcohol

The product obtained in example 3 was treated with 30% by weight of ethanol. The system was kept under stirring, observing the precipitation of a solid product that was centrifuged off. The liquid product obtained was subjected to evaporation under vacuum, to remove the ethanol still present, while the separated solid was reused in a subsequent hydrolysis reaction (example 5). The product was characterised in terms of acidity and the amount of zinc still present after the separation from the catalyst was determined (table 5).

**Table 5. Characterisation of ester after treatment with ethanol.**

| **Parameter** | **Ester** |
|---|---|
| **Acid number after separation of ZnO [mg KOH/g sample]** | 2.57 |
| **Zn [ppm]** | 70.26 |

### Example 5 - Reuse in hydrolysis of solid from example 4 as a catalyst

A hydrolysis reaction was carried out, loading in an autoclave 150 g of used frying oil (with the same characteristics as those used in example 2), 45 g of water, corresponding to 30% by weight with respect to oil and zinc oxide separated from the product obtained from the previous test, as described in example 4. Heating was started up to a temperature of 200°C, reaching a maximum pressure of 13,6 bar. The reaction had a total duration of 7 hours, at the end of which the system was cooled at a temperature of about 60°C and fatty acids produced were separated from the aqueous phase containing glycerol. Fatty acids produced were characterised, obtaining the results reported in table 6.

**Table 6. Characterisation of fatty acids.**

| **Parameter** | **Ester** |
|---|---|
| **Acid number [mg KOH/g sample]** | 156.34 |
| **Iodine number [g I₂/100g sample]** | 64.24 |

### Example 6 - Multiple step treatment of product from example 4

The product obtained as described in example 3 was treated in multiple steps. Each step has the same procedure: the product is placed under stirring with the solvent used in the treatment and subsequently the solid precipitate is separated by centrifugation. At the end of the treatments, the product obtained undergoes evaporation under vacuum, to remove the still present solvent. Hereinafter the different treatments carried out and the results of the analyses after each treatment for the determination of the amount of residual zinc (table 7) are reported.

Treatment 1: single treatment with ethanol in an amount equal to 30% by weight with respect to the ester (example 4).

Treatment 2: double treatment with ethanol in an amount equal to 30% by weight with respect to the ester.

Treatment 3: double treatment with ethanol in an amount equal to 30% by weight with respect to the ester, followed by a single treatment with H₂O in an amount equal to 30% by weight with respect to the ester.

Treatment 4: double treatment with ethanol in an amount equal to 30% by weight with respect to the ester, followed by a single treatment with Amberlyst-15 in an amount equal to 5% by weight with respect to the ester.

Treatment 5: single treatment with ethanol in an amount equal to 30% by weight with respect to the ester, followed by a single treatment with H₂O in an amount equal to 30% by weight with respect to the ester.

Treatment 6: single treatment with ethanol in an amount equal to 30% by weight with respect to the ester, followed by a double treatment with H₂O in an amount equal to 30% by weight with respect to the ester.

Treatment 7: single treatment with ethanol in an amount equal to 30% by weight with respect to the ester, followed by a single treatment with Amberlyst-15 in an amount equal to 10% by weight with respect to the ester.

Treatment 8: single treatment with ethanol in an amount equal to 30% by weight with respect to the ester, followed by a single treatment with Amberlyst-15 in an amount equal to 10% by weight with respect to the ester and H₂O in an amount equal to 30% by weight with respect to the ester.

Treatment 9: single treatment with ethanol in an amount equal to 30% by weight with respect to the ester, followed by a single treatment with clay (Tonsil EX2166) in an amount equal to 10% by weight with respect to the ester.

Treatment 10: single treatment with ethanol in an amount equal to 30% by weight with respect to the ester, followed by a single treatment with H₂O in an amount equal to 30% by weight with respect to the ester and Amberlyst-15 in an amount equal to 10% by weight with respect to the ester.

Treatment 11: single treatment with ethanol in an amount equal to 30% by weight with respect to the ester, followed by a single treatment with H₂O in an amount equal to 30% by weight with respect to the ester and clay (Tonsil EX2166) in an amount equal to 10% by weight with respect to the ester.

Treatment 12: single treatment with ethanol in an amount equal to 30% by weight with respect to the ester, followed by a single treatment with H₂O in an amount equal to 30% by weight with respect to the ester and clay (Tonsil EX096) in an amount equal to 10% by weight with respect to the ester.

**Table 7. Residual zinc after carrying out each treatment.**

| **Treatment** | | **Zn [ppm]** |
|---|---|---|
| | 1 | 70.26 |
| | 2 | 51.07 |
| | 3 | 26.11 |
| | 4 | 33.60 |
| | 5 | 26.05 |
| | 6 | 13.71 |
| | 7 | 3.05 |
| | 8 | 1.32 |
| | 9 | 22.1 |
| | 10 | 0.82 |
| | 11 | 2.67 |
| | 12 | 0.92 |

Anyway, it is understood that the invention should not be considered limited to the particular arrangement illustrated above, which constitutes only an exemplary embodiment thereof, but different changes are possible, all within the reach of the skilled persons, without thereby departing from the scope of protection of the invention itself, as defined by the following claims.

### LIST OF REFERENCE NUMERALS

1 feed (of 2) of waste lubricants
2 hydrolysis reactor
3 feed (of 2) of water
4 stirrer (of 2)
5 feeding flow of hydrolysis catalyst
6 flow from 2 to 7
7 esterification reactor
8 acidification reactor
9 stirrer (of 8)
10 feed (of 8) of sulphuric acid
11 feed (of 8) of waste soaps
12 flow from 8 to 7
13 feed (of 7) of zinc oxide
14 feed (of 7) of waste fatty acids
15 feed (of 7) of alcohols
16 three-way valve (of 15)
17 feed of monoalcohols
18 feed of polyalcohols
19 stirrer (of 7)
20 flow from 7 to 21
21 precipitation reactor
22 stirrer (of 21)
23 feed (of 21) of non-solvent
24 exit flow from 22
25 separator
26 product

## Claims

1. Process for the production of solvents and/or lubricants from renewable sources, comprising the steps of fat hydrolysis and an esterification reaction of fatty acids with alcohols in the presence of zinc oxide, **characterised in that** to the product of the fatty acid esterification reaction, a liquid substance in which zinc oxide is not capable to dissolve, generally called non-solvent, is added, obtaining a precipitate that is separated from the product and used as a catalyst in the previous hydrolysis step of the fats used as a raw material.

2. Process as in claim 1), **characterised in that** a resin or clay is added together with the non-solvent.

3. Process as in claims 1) or 2), **characterised in that** said non-solvent is selected from the group consisting of water, alcohols, such as methanol, ethanol, propanol or butanol, and mixtures thereof.

4. Process as in any one of the preceding claims, **characterised in that** said non-solvent is added in a stoichiometric amount.

5. Process as in any one of claims 1) to 3), **characterised in that** said non-solvent is added in excess with respect to the stoichiometric amount.

6. Process as in any one of the preceding claims, **characterised in that** it also provides an acidification step of waste soaps and surfactants.

7. Process as in any one of the preceding claims, **characterised in that** said precipitate used as a catalyst in the fat hydrolysis step is used as a catalyst also in the esterification reaction of fatty acids.

8. Process as in any one of the preceding claims, **characterised in that** the esterification reaction of fatty acids is carried out by reacting fatty acids with an alcohol selected from the group consisting of 2-ethyl-hexanol, ethylene glycol, propylene glycol, 1,4-butylene glycol, 1,3-butylene glycol, neopentyl glycol, trimethylolpropane, pentaerythritol.

9. Process as in any one of the preceding claims, **characterised in that** fatty acids that undergo the esterification reaction have a number of carbon atoms ranging between 8 and 22.

10. Process as in any one of the preceding claims, **characterised in that** the treatment with a non-solvent is carried out several times.

## Patentansprüche

1. Verfahren zur Herstellung von Lösungsmitteln und/oder Schmiermitteln aus erneuerbaren Quellen, umfassend die Schritte der Fetthydrolyse und einer Veresterungsreaktion von Fettsäuren mit Alkoholen in Gegenwart von Zinkoxid, **dadurch gekennzeichnet, dass** dem Produkt der Fettsäureveresterungsreaktion eine flüssige Substanz, in der sich Zinkoxid nicht lösen kann, im Allgemeinen Nichtlösungsmittel genannt, zugegeben wird, wobei ein Niederschlag erhalten wird, der von dem Produkt abgetrennt wird und als Katalysator in dem vorhergehenden Hydrolyseschritt der als Rohmaterial verwendeten Fette verwendet wird.

2. Verfahren nach Anspruch 1), **dadurch gekennzeichnet, dass** ein Harz oder Ton zusammen mit dem Nichtlösungsmittel zugegeben wird.

3. Verfahren nach den Ansprüchen 1) oder 2), **dadurch gekennzeichnet, dass** das Nichtlösungsmittel aus der Gruppe ausgewählt ist, die aus Wasser, Alkoholen, wie Methanol, Ethanol, Propanol oder Butanol, und Gemischen davon besteht.

4. Verfahren nach einem der vorhergehenden Ansprüche, **dadurch gekennzeichnet, dass** das Nichtlösungsmittel in einer stöchiometrischen Menge zugegeben wird.

5. Verfahren nach einem der Ansprüche 1) bis 3), **dadurch gekennzeichnet, dass** das Nichtlösungsmittel im Überschuss in Bezug auf die stöchiometrische Menge zugegeben wird.

6. Verfahren nach einem der vorhergehenden Ansprüche, **dadurch gekennzeichnet, dass** es auch einen Ansäuerungsschritt von Abfallseifen und Tensiden bereitstellt.

7. Verfahren nach einem der vorhergehenden Ansprüche, **dadurch gekennzeichnet, dass** der Niederschlag, der als Katalysator in dem Fetthydrolyseschritt verwendet wird, auch als Katalysator in der Veresterungsreaktion von Fettsäuren verwendet wird.

8. Verfahren nach einem der vorhergehenden Ansprüche, **dadurch gekennzeichnet, dass** die Veresterungsreaktion von Fettsäuren durch Umsetzen von Fettsäuren mit einem Alkohol durchgeführt wird, der aus der Gruppe ausgewählt ist, die aus 2-Ethylhexanol, Ethylenglycol, Propylenglycol, 1,4-Butylenglycol, 1,3-Butylenglycol, Neopentylglycol, Trimethylolpropan, und Pentaerythrit besteht.

9. Verfahren nach einem der vorhergehenden Ansprüche, **dadurch gekennzeichnet, dass** die Fettsäuren, die der Veresterungsreaktion unterzogen werden, eine Anzahl von Kohlenstoffatomen aufweisen, die zwischen 8 und 22 liegt.

10. Verfahren nach einem der vorhergehenden Ansprüche, **dadurch gekennzeichnet, dass** die Behandlung mit einem Nichtlösungsmittel mehrmals durchgeführt wird.

## Revendications

1. Procédé de production de solvants et/ou de lubrifiants à partir de sources renouvelables, comprenant les étapes d'hydrolyse des graisses et une réaction d'estérification d'acides gras avec des alcools en présence d'oxyde de zinc, **caractérisé en ce qu'**une substance liquide dans laquelle l'oxyde de zinc n'est pas capable de se dissoudre, généralement appelée non-solvant, est ajoutée au produit de la réaction d'estérification d'acides gras, pour obtenir un précipité qui est séparé du produit et utilisé comme catalyseur dans l'étape précédente d'hydrolyse des graisses utilisées comme matière première.

2. Procédé selon la revendication 1), **caractérisé en ce qu'**une résine ou une argile est ajoutée conjointement avec le non-solvant.

3. Procédé selon les revendications 1) ou 2), **caractérisé en ce que** ledit non-solvant est sélectionné dans le groupe consistant en l'eau, les alcools tels que le méthanol, l'éthanol, le propanol ou le butanol, et les mélanges de ceux-ci.

4. Procédé selon l'une des revendications précédentes, **caractérisé en ce que** ledit non-solvant est ajouté en quantité stœchiométrique.

5. Procédé selon l'une des revendications 1) à 3), **caractérisé en ce que** ledit non-solvant est ajouté en excès par rapport à la quantité stœchiométrique.

6. Procédé selon l'une des revendications précédentes, **caractérisé en ce qu'**il prévoit également une étape d'acidification des savons et tensioactifs résiduaires.

7. Procédé selon l'une des revendications précédentes, **caractérisé en ce que** ledit précipité utilisé comme catalyseur dans l'étape d'hydrolyse des graisses est utilisé comme catalyseur également dans la réaction d'estérification des acides gras.

8. Procédé selon l'une des revendications précédentes, **caractérisé en ce que** la réaction d'estérification des acides gras est réalisée en faisant réagir les acides gras avec un alcool sélectionné dans le groupe consistant en le 2-éthyl-hexanol, l'éthylèneglycol, le propylèneglycol, le 1,4-butylèneglycol, le 1,3-butylèneglycol, le néopentylglycol, le triméthylolpropane, le pentaérythritol.

9. Procédé selon l'une des revendications précédentes, **caractérisé en ce que** les acides gras qui subissent la réaction d'estérification possèdent un nombre d'atomes de carbone entre 8 et 22.

10. Procédé selon l'une des revendications précédentes, **caractérisé en ce que** le traitement avec un non-solvant est effectué plusieurs fois.
